(19) Europäisches Patentamt
European Patent Office
Office européen des brevets

(11) Veröffentlichungsnummer: **0 095 696**
**B1**

(12) # EUROPÄISCHE PATENTSCHRIFT

(45) Veröffentlichungstag der Patentschrift: **10.04.85**

(21) Anmeldenummer: **83105088.5**

(22) Anmeldetag: **24.05.83**

(51) Int. Cl.⁴: **C 07 C 61/39**, C 07 C 61/40, C 07 C 62/34, C 07 C 69/753, C 07 C 69/757, C 07 D 333/24, C 07 D 307/54, C 07 C 51/00, C 07 C 67/00, C 07 C 49/233

(54) **Verfahren zur Herstellung von 2,2-Dimethyl-3-arylcyclopropan-carbonsäuren (carbonsäureestern), neue Zwischenprodukte hierfür und Verfahren zu deren Herstellung.**

(30) Priorität: **02.06.82 DE 3220732**

(43) Veröffentlichungstag der Anmeldung: **07.12.83 Patentblatt 83/49**

(45) Bekanntmachung des Hinweises auf die Patenterteilung: **10.04.85 Patentblatt 85/15**

(84) Benannte Vertragsstaaten: **BE CH DE FR GB LI**

(56) Entgegenhaltungen:
**EP - A - 0 043 492**
**EP - A - 0 056 154**
**DD - A - 157 792**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Arlt, Dieter, Prof. Dr., Rybnikerstrasse 2, D-5000 Köln 80 (DE)**
Erfinder: **Jautelat, Manfred, Dr., Müllersbaum 28, D-5093 Burscheid (DE)**

EP 0 095 696 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von weitgehend bekannten 2,2-Dimethyl-3-arylcyclopropancarbonsäuren (-carbonsäureestern), welche als Zwischenprodukte zur Herstellung von insektizid wirksamen 2,2-Dimethyl-3-arylcyclopropancarbonsäureestern verwendet werden können, neue 1-Aryl-1,4-dihalogen-2,2-dimethyl-3-butanone als Zwischenprodukte hierfür und ein Verfahren zu deren Herstellung sowie neue 1-Aryl-1-halogen-2,2-dimethyl-3-butanone als Zwischenprodukte hierfür und ein Verfahren zu deren Herstellung.

Es ist bekannt, dass man bestimmte 2,2-Dimethyl-3-arylcyclopropancarbonsäuren erhält, wenn man entsprechende 1-Aryl-2-methylpropene mit Diazoessigsäureestern in Gegenwart von Katalysatoren umsetzt und die hierbei gebildeten 2,2-Dimethyl-3-arylcyclopropancarbonsäureester hydrolysiert (vgl. „Coll. Czech. Chem. Commun.", *25* (1960), 1815). Diese Synthesemethode besitzt jedoch nur einen begrenzten Anwendungsbereich. Die Ausbeuten sind stark von der Art der Arylreste abhängig und in vielen Fällen unbefriedigend; bei der Umsetzung von 1-Thienyl-2-methylpropenen mit Diazoessigsäureestern werden nur Zersetzungsprodukte erhalten. Die Verwendung von Diazoessigsäureestern ist zudem mit den bekannten Sicherheitsrisiken behaftet. Ferner lassen sich die Ausgangsstoffe meist nur schwierig herstellen.

Weiter ist die Synthese von 2,2-Dimethyl-3-phenylcyclopropancarbonsäureethylester über 4-Methyl-3-phenylvalerolacton bekannt geworden (vgl. „Bull. Soc. Chim. France", *1961*, 1857). Dieser Weg erfordert jedoch eine Reihe von zum Teil relativ komplizierten Vorstufen und liefert insgesamt nur eine geringe Ausbeute.

2,2-Dimethyl-3-phenylcyclopropancarbonsäureester erhält man auch durch katalytische Hydrierung von 2,2-Dimethyl-3-phenylcyclopropencarbonsäureestern (vgl. „Chem. Ber.", *111* (1978), 3879). Die Synthese der als Ausgangsstoffe benötigten 2,2-Dimethyl-3-phenylcyclopropencarbonsäureester verläuft jedoch über mehrere, zum Teil sehr schwierige Stufen.

Ferner können 2,2-Dimethyl-3-arylcyclopropancarbonsäureester auch durch Umsetzung von Zimtsäureestern mit Triphenylisopropylphosphoniumiodid in Gegenwart starker Basen, wie z.B. Butyllithium, hergestellt werden (vgl. JP Nr. 8105435). Dieses Verfahren ist jedoch technisch aufwendig und teuer.

Die vorliegende Erfindung betrifft

1) ein Verfahren zur Herstellung von 2,2-Dimethyl-3-arylcyclopropancarbonsäuren (-carbonsäureestern) der Formel I

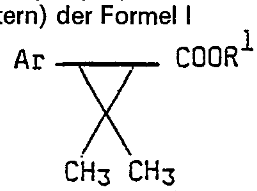

(I)

in welcher

$R^1$ für H oder $C_1$-$C_4$-Alkyl steht,

Ar für Naphthyl oder für den Rest

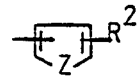

steht, worin

Z für Sauerstoff, Schwefel oder Ethen-1,2-diyl (−CH=CH−) steht,

$R^2$ für Wasserstoff, Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht,

dadurch gekennzeichnet, dass man 1-Aryl-1,4-dihalogen-2,2-dimethyl-3-butanone der Formel II

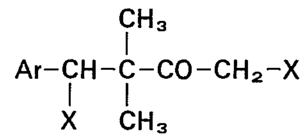

(II)

in welcher

Ar die oben angegebene Bedeutung hat, und

X für Chlor oder Brom steht,

mit Basen aus der Reihe der Alkalimetall- und Erdalkalimetallhydroxide, -alkoholate und -carbonate in Gegenwart von Wasser und organischer Lösungsmittel gegebenenfalls in Gegenwart von Katalysatoren bei Temperaturen zwischen −20 und +150 °C umsetzt;

2) neue 1-Aryl-1,4-dihalogen-2,2-dimethyl-3-butanone der Formel II

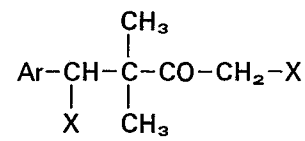

(II)

in welcher

X für Chlor oder Brom steht, und

Ar für Naphthyl oder für den Rest

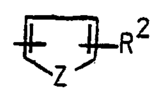

steht,

worin

Z für Sauerstoff, Schwefel oder Ethen-1,2-diyl (−CH=CH−) steht, und

$R^2$ für Wasserstoff, Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht;

3) ein Verfahren zur Herstellung der neuen 1-Aryl-1,4-dihalogen-2,2-dimethyl-3-butanone der Formel II (oben), dadurch gekennzeichnet, dass man 1-Aryl-1-halogen-2,2-dimethyl-3-butanone der Formel III

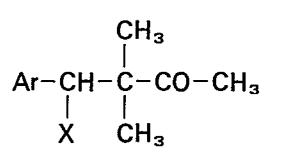

(III)

in welcher

Ar und X die oben angegebene Bedeutung haben,

mit Halogenen (Chlor oder Brom) in Gegenwart inerter Verdünnungsmittel bei Temperaturen zwischen −30 und +50 °C umsetzt;

4) neue 1-Aryl-1-halogen-2,2-dimethyl-3-butanone der Formel IIIa

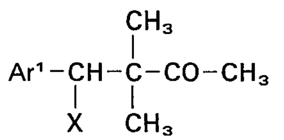

(IIIa)

in welcher

X für Chlor oder Brom steht, und

$Ar^1$ für Naphthyl oder für den Rest

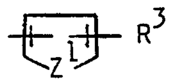

steht,

worin

$Z^1$ für Sauerstoff oder Ethen-1,2-diyl (−CH=CH−) steht, und

$R^3$ für Cyano, Nitro, Trialkylsilyl oder für einen gegebenenfalls durch Halogen substituierten Rest aus der Reihe Cylcoalkyl, Alkenyl, Alkylendioxy, Alkylthio, Alkylsulfinyl, Alkylsulfonyl, Dialkylamino, Phenyl und Phenoxy, oder für von Methyl verschiedenes Alkyl, oder für von Methoxy verschiedenes Alkoxy, oder für Halogenalkyl oder Halogenalkoxy steht, oder, für den Fall, dass X für Brom steht, auch für Wasserstoff, Halogen, Methyl oder Methoxy steht;

5) ein Verfahren zur Herstellung der neuen 1-Aryl-1-halogen-2,2-dimethyl-3-butanone der Formel IIIa (oben), dadurch gekennzeichnet, dass man 1-Aryl-2,2-dimethyl-3-butanone der Formel IV

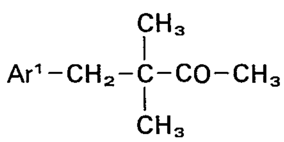

(IV)

in welcher

$Ar^1$ die oben angegebene Bedeutung hat,

gegebenenfalls in Gegenwart eines inerten Verdünnungsmittels und in Gegenwart eines Katalysators bei Temperaturen zwischen 40 und 120 °C radikalisch halogeniert.

Es ist als überraschend anzusehen, dass man ausgehend von 1-Aryl-2,2-dimethyl-3-butanonen über die entsprechenden 1-Halogen- und 1,4-Dihalogenderivate 2,2-Dimethyl-3-arylcyclopropancarbonsäuren (-carbonsäureester) in guten Ausbeuten herstellen kann, da aus der Literatur bekannt ist, dass 1-Aryl-1-halogen-2,2-dimethyl-3-butanone mit Alkalimetallhydroxiden in wässerig-organischer Lösung unter Substitution des Halogens gegen eine Hydroxygruppe reagieren (vgl. „Arch. Pharm.", *313* (1980), 795). Ebenso war nicht vorauszusehen, dass einerseits die radikalische Halogenierung der 1-Aryl-2,2-dimethyl-3-butanone in 1-Stellung (an der Methylengruppe) praktisch ohne konkurrierenden Angriff des Halogenierungsmittels in 4-Stellung (an der Methylgruppe) möglich ist und andererseits die Reaktion der hierbei gebildeten 1-Aryl-1-halogen-2,2-dimethyl-3-butanone mit elementarem Halogen selektiv an der Methylgruppe unter Bildung der 1-Aryl-1,4-dihalogen-2,2-dimethyl-3-butanone erfolgen werde.

Das erfindungsgemässe Verfahren weist eine Reihe von Vorteilen auf. So können preiswerte bzw. leicht herzustellende Reaktionskomponenten und Lösungsmittel ohne besondere Vorbehandlung eingesetzt werden. Die einzelnen Verfahrensschritte können ohne besonderen Aufwand durchgeführt werden und man erhält die Produkte im allgemeinen in hohen Ausbeuten.

Verwendet man bei dem oben unter 1 dargelegten Verfahren zur Herstellung von 2,2-Dimethyl-3-arylcyclopropancarbonsäuren (-carbonsäureestern) beispielsweise 1-(4-Fluorphenyl)-1,4-dichlor-2,2-dimethyl-3-butanon und Kaliumhydroxid als Ausgangskomponenten, so kann an diesem Beispiel das erfindungsgemässe Verfahren durch folgendes Formelschema skizziert werden

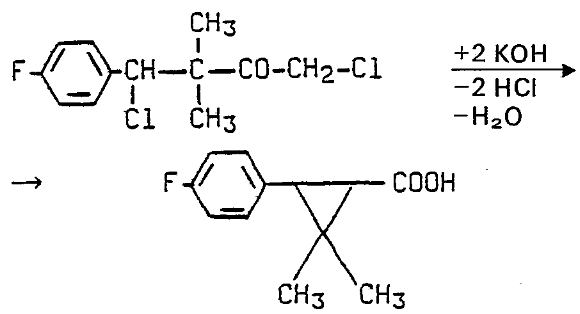


Zur Durchführung des oben unter 1 dargelegten erfindungsgemässen Verfahrens werden neben Wasser organische Lösungsmittel eingesetzt. Als solche kommen vorzugsweise einerseits polare, mit Wasser mischbare Solvenzien, wie z.B. Aceton, Methylethylketon, Methylisobutylketon, Acetonitril, Tetrahydrofuran, Dioxan und 1,2-Dimethoxyethan, oder andererseits mit Wasser praktisch nicht mischbare Lösungsmittel aus der Reihe der gegebenenfalls halogenierten Kohlenwasserstoffe, wie z.B. Hexan, Cyclohexan, Heptan, Petrolether, Ligroin, Methylenchlorid, Chloroform, Benzol, Toluol und Xylol in Betracht.

Ausserdem können auch Alkohole als Lösungsmittel eingesetzt werden, wie z.B. Methanol, Ethanol, Isopropanol und tert.-Butanol.

Bei Verwendung von mit Wasser praktisch nicht mischbaren Lösungsmitteln werden vorzugsweise Phasentransferkatalysatoren aus der Reihe der Tetraalkyl- oder Trialkylaralkylammoniumsalze, wie z.B. Tetrabutylammoniumbromid oder Triethylbenzylammoniumchlorid eingesetzt.

Als Basen werden beim erfindungsgemässen Verfahren Alkalimetall- oder Erdalkalimetallhydroxide, wie z.B. Natrium-, Kalium-, Magnesium- und Calciumhydroxid, Alkalimetall- oder Erdalkalimetallcarbonate, wie z.B. Natrium-, Kalium-, Magnesium- und Calciumcarbonat oder Alkalialkoholate, wie z.B. Natrium- oder Kaliummethylat, Natrium-oder Kaliumethylat, Natriumisopropylat und Kalium-tert.-butylat eingesetzt.

Zur Herstellung der Säuren werden vorzugsweise Alkalimetallhydroxide, wie z.B. Natriumhydroxid oder Kaliumhydroxid eingesetzt.

Zur Herstellung der Ester werden vorzugsweise die Alkalialkoholate, wie z.B. Natriummethylat, Natriumethylat, Natriumisopropylat und Kalium-tert.-butylat eingesetzt. Als Lösungsmittel verwendet man dann vorzugsweise die entsprechenden Alkohole.

Die Reaktionstemperaturen werden bei der Durchführung des erfindungsgemässen Verfahrens im allgemeinen zwischen −20 und +150 °C, vorzugsweise zwischen +10 und +100 °C gehalten.

Das erfindungsgemässe Verfahren wird im allgemeinen bei Normaldruck durchgeführt.

Zur Durchführung des erfindungsgemässen Verfahrens setzt man im allgemeinen je 1 mol 1-Aryl-1,4-dihalogen-2,2-dimethyl-3-butanon 2 bis 15, vorzugsweise 2 bis 10 Mol-Eq einer Base ein. In einer bevorzugten Ausführungsform des erfindungsgemässen Verfahrens wird die wässerige Lösung der Base, gegebenenfalls verdünnt mit einem organischen Lösungsmittel, vorgelegt und das 1-Aryl-1,4-dihalogen-2,2-dimethyl-3-butanon der Formel II, gegebenenfalls in einem organischen Lösungsmittel gelöst, und gegebenenfalls ein Katalysator dazu gegeben. Das Reaktionsgemisch wird bis zum Ende der Umsetzung gerührt und auf übliche Weise, beispielsweise durch Ansäuern und Extrahieren mit Methylenchlorid, aufgearbeitet. Das Produkt der Formel I erhält man nach Abdestillieren des organischen Extraktionsmittels als kristallinen Rückstand.

Die als Zwischenprodukte beim erfindungsgemässen Verfahren zu verwendenden neuen 1-Aryl-1,4-dihalogen-2,2-dimethyl-3-butanone sind durch die Formel II – oben unter 2 – allgemein definiert.

In dieser Formel stehen vorzugsweise

X für Chlor oder Brom, und

Ar für Naphthyl oder für den Rest

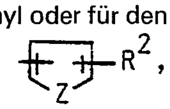,

worin

Z für Sauerstoff, Schwefel oder Ethen-1,2-diyl (−CH=CH−) steht, und

$R^2$ für Wasserstoff, Fluor, Chlor, Brom, Cyano, Nitro, Trimethylsilyl oder für einen gegebenenfalls durch Chlor und/oder Fluor substituierten Rest aus der Reihe $C_1$-$C_6$-Alkyl, $C_3$-$C_6$-Cycloalkyl, $C_2$-$C_3$-Alkenyl, $C_1$-$C_4$-Alkoxy, $C_1$-$C_2$-Alkylendioxy, $C_1$-$C_4$-Alkylthio, $C_1$-$C_4$-Alkylsulfinyl, $C_1$-$C_4$-Alkylsulfonyl, Di-$C_1$-$C_4$-alkylamino, Phenyl und Phenoxy steht.

Besonders bevorzugt sind die Verbindungen der Formel II, in welcher

X für Chlor oder Brom steht, und

Ar für Phenyl steht, welches gegebenenfalls in meta- und/oder para-Position durch Fluor, Chlor, Brom, $C_1$-$C_4$-Alkyl, Trifluormethyl, Methoxy, Trifluormethoxy, Methylendioxy oder Difluormethylendioxy substituiert ist.

Als Beispiele für die neuen Verbindungen der Formel II seien genannt

1-Phenyl-, 1-(4-Fluorphenyl)-, 1-(4-Chlorphenyl)-, 1-(4-Methylphenyl)-, 1-(4-tert.-Butylphenyl)-, 1-(4-Trifluormethylphenyl)-, 1-(4-Methoxyphenyl)- und 1-(4-Trifluormethoxyphenyl)-1,4-dichlor-2,2-dimethyl-3-butanon sowie 1-Phenyl-, 1-(4-Fluorphenyl)-, 1-(4-Chlorphenyl)-, 1-(4-Methylphenyl)-, 1-(4-tert.-Butylphenyl)-, 1-(4-Trifluormethylphenyl)-, 1-(4-Methoxyphenyl)- und 1-(4-Trifluormethoxyphenyl)-1,4-dibrom-2,2-dimethyl-3-butanon.

Verwendet man bei dem unter 3 dargelegten Verfahren zur Herstellung der neuen 1-Aryl-1,4-dihalogen-2,2-dimethyl-3-butanone beispielsweise 1-(4-Fluorphenyl)-1-chlor-2,2-dimethyl-3-butanon und Chlor als Ausgangsstoffe, so kann deren erfindungsgemässe Umsetzung durch folgendes Formelschema skizziert werden

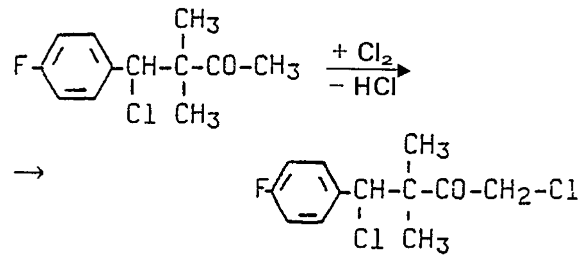


Das unter 3 dargelegte erfindungsgemässe Verfahren wird unter Verwendung inerter Verdünnungsmittel durchgeführt. Als solche kommen vorzugsweise halogenierte Kohlenwasserstoffe, wie z.B. Methylenchlorid, Chloroform, Dichlordifluormethan, Tetrachlormethan und Ethylenchlorid, und gegebenenfalls auch Carbonsäuren, wie z.B. Essigsäure, in Betracht.

Die Umsetzung wird bei Temperaturen zwischen −30 und +50 °C, vorzugsweise bei 0 bis +20 °C und im allgemeinen bei Normaldruck durchgeführt.

Auf 1 mol 1-Aryl-1-halogen-2,2-dimethyl-3-butanon der Formel III werden im allgemeinen 0,8 bis 1,2 mol, vorzugsweise 0,9 bis 1 mol Chlor oder Brom eingesetzt.

In einer bevorzugten Ausführungsform des unter 3 dargelegten Verfahrens wird eine Lösung des 1-Aryl-1-halogen-2,2-dimethyl-3-butanons vorgelegt und das Halogen wird gasförmig oder in Lösung langsam eindosiert. Nach Ende der Umsetzung wird das Produkt der Formel II durch Abdestillieren des Lösungsmittels isoliert.

Die als Zwischenprodukte benötigten 1-Aryl-1-halogen-2,2-dimethyl-3-butanone sind durch die Formel III – oben unter 3 – allgemein definiert. In Formel III haben die Reste Ar und X vorzugsweise bzw. besonders bevorzugt die gleichen Bedeutungen, wie sie bei der Definition der entsprechenden Reste Ar und X in Formel II als vorzugsweise bzw. besonders bevorzugt angegeben sind.

Als Beispiele für bereits bekannte Verbindungen der Formel III, vgl. „Arch. Pharm.", *308* (1975), 422, seien genannt 1-(4-Methylphenyl)-, 1-(4-Methoxyphenyl)- und 1-(2-Thienyl)-1-chlor-2,2-dimethyl-3-butanon.

Die neuen 1-Aryl-1-halogen-2,2-dimethyl-3-butanone sind durch die Formel IIIa – oben unter 4 – definiert. In dieser Formel stehen vorzugsweise

X für Chlor oder Brom, und

$Ar^1$ für Naphthyl oder für den Rest

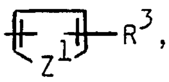

worin

$Z^1$ für Sauerstoff oder Ethen-1,2-diyl (–CH=CH–) steht, und

$R^3$ für Fluor, Brom, Cyano, Nitro, Trimethylsilyl, für durch Fluor und/oder Chlor substituierte Reste Methyl oder Methoxy oder für gegebenenfalls durch Fluor und/oder Chlor substituierte Reste aus der Reihe $C_2$-$C_6$-Alkyl, $C_3$-$C_6$-Cycloalkyl, $C_2$-$C_6$-Alkenyl, $C_2$-$C_4$-Alkoxy, $C_1$-$C_2$-Alkylendioxy, $C_1$-$C_4$-Alkylthio, $C_1$-$C_4$-Alkylsulfinyl, $C_1$-$C_4$-Alkylsulfonyl, Di-$C_1$-$C_4$-alkylamino, Phenyl und Phenoxy, oder, für den Fall, dass X für Brom steht, auch für Wasserstoff, Chlor, Methyl oder Methoxy stehen.

Besonders bevorzugt sind die neuen Verbindungen der Formel IIIa, in welcher

X für Chlor oder Brom steht, und

$Ar^1$ für Phenyl steht, welches in meta- und/oder para-Position durch Fluor, Brom, $C_2$-$C_4$-Alkyl, Trifluormethyl, Trifluormethoxy, Methylendioxy oder Difluormethylendioxy substituiert ist, und, für den Fall, dass X für Brom steht, auch für Phenyl oder für durch Methyl, Methoxy oder Chlor in meta- und/oder para-Position substituiertes Phenyl steht.

Als Beispiele für die neuen Verbindungen der Formel IIIa seien genannt

1-(4-Fluorphenyl)-, 1-(4-tert.-Butylphenyl)-, 1-(4-Trifluormethylphenyl)- und 1-(4-Trifluormethoxyphenyl)-1-chlor-2,2-dimethyl-3-butanon sowie 1-Phenyl-, 1-(4-Fluorphenyl)-, 1-(4-Chlorphenyl)-, 1-(4-Methylphenyl)-, 1-(4-tert.-Butylphenyl)-, 1-(4-Trifluormethylphenyl)-, 1-(4-Methoxyphenyl)- und 1-(4-Trifluormethoxyphenyl)-1-brom-2,2-dimethyl-3-butanon.

Verwendet man bei dem oben unter 5 dargelegten Verfahren zur Herstellung der neuen 1-Aryl-1-halogen-2,2-dimethyl-3-butanone der Formel IIIa beispielsweise 1-(4-Fluorphenyl)-2,2-dimethyl-3-butanon und N-Chlorsuccinimid als Ausgangsstoffe, so kann deren erfindungsgemässe Umsetzung durch folgendes Formelschema skizziert werden

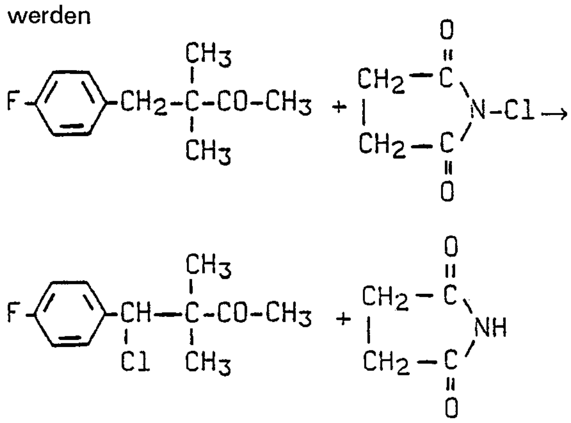

Das unter 5 dargelegte erfindungsgemässe Verfahren wird vorzugsweise unter Verwendung inerter Verdünnungsmittel durchgeführt. Als solche kommen vorzugsweise halogenierte Kohlenwasserstoffe, wie z.B. Methylenchlorid, Chloroform, Dichlordifluormethan, Tetrachlormethan und Ethylenchlorid in Betracht.

Als Katalysatoren werden vorzugsweise freie Radikale liefernde Verbindungen, wie z.B. Azobisisobutyronitril, Benzoylperoxid oder Di-tert.-butylperoxid verwendet.

Die Umsetzung wird im allgemeinen bei Temperaturen zwischen 40 und 120 °C, vorzugsweise bei 60 bis 100 °C und im allgemeinen bei Normaldruck oder mässig erhöhtem Druck bei etwa 10 bar durchgeführt.

Auf 1 mol 1-Aryl-2,2-dimethyl-3-butanon der Formel IV setzt man im allgemeinen zwischen 1 und 2 Mol-Eq, vorzugsweise 1 bis 1,2 Mol-Eq eines Halogenierungsmittels ein. Geeignete Halogenierungsmittel sind N-Halogen-succinimide, wie z.B. N-Bromsuccinimid oder N-Chlorsuccinimid.

Zur Durchführung des unter 5 dargelegten Verfahrens zur Herstellung der neuen 1-Aryl-1-halogen-2,2-dimethyl-3-butanone der Formel IIIa werden entsprechende 1-Aryl-2,2-dimethyl-3-butanone, Halogenierungsmittel und Katalysatoren in geeigneten Verdünnungsmitteln bis zum Ende der Umsetzung erhitzt und die Produkte der Formel IIIa werden durch Hochvakuumdestillation isoliert.

Das unter 5 dargelegte Verfahren ist auch zur Herstellung von bekannten Verbindungen der Formel III, wie z.B. von 1-(4-Chlorphenyl)-1-chlor-2,2-dimethyl-3-butanon, wesentlich besser geeignet als die aus der Literatur bekannte Synthesemethode (vgl. „Arch. Pharm.", *308* (1975), 422).

Die als Zwischenprodukte benötigten 1-Aryl-2,2-dimethyl-3-butanone sind durch die Formel IV – oben unter 5 – allgemein definiert. In Formel IV haben die Reste $Ar^1$ und X vorzugsweise bzw. besonders bevorzugt die gleichen Bedeutungen, wie sie oben bei der Definition der entsprechenden Reste $Ar^1$ und X in Formel IIIa als vorzugsweise bzw. besonders bevorzugt angegeben sind.

Als Beispiele für die Verbindungen der Formel IV seien genannt

1-Phenyl-, 1-(4-Fluorphenyl)-, 1-(4-Chlorphenyl)-, 1-(4-Methylphenyl)-, 1-(4-tert.-Butylphenyl)-, 1-(4-Trifluormethylphenyl)-, 1-(4-Methoxyphenyl)- und 1-(4-Trifluormethoxyphenyl)-2,2-dimethyl-3-butanon.

Die 1-Aryl-2,2-dimethyl-3-butanone der Formel IV sind zum Teil noch nicht in der Literatur beschrieben. Ein Verfahren zu ihrer Herstellung ist Gegenstand einer nicht vorveröffentlichten Patentanmeldung.

Man erhält demnach die 1-Aryl-2,2-dimethyl-3-butanone der Formel IV, wenn man Arylmethylhalogenide der Formel V

$$Ar^1-CH_2-X^1 \qquad (V)$$

in welcher

$Ar^1$ die oben angegebene Bedeutung hat, und $X^1$ für Halogen, vorzugsweise für Chlor, steht, mit Methylisopropylketon in Gegenwart einer Base, wie z.B. Kaliumhydroxid, in Gegenwart eines Verdünnungsmittels, wie z.B. Toluol, und in Gegenwart eines Phasentransferkatalysators, wie z.B. Tetrabutylammoniumbromid, bei Temperaturen zwischen 20 und 130 °C umsetzt und nach Filtrieren und Waschen der Reaktionsmischung die Produkte der Formel IV durch Hochvakuumdestillation isoliert.

Die nach dem erfindungsgemässen Verfahren herzustellenden 2,2-Dimethyl-3-arylcyclopropancarbonsäuren der Formel I können als Zwischenprodukte zur Herstellung von insektizid wirksamen 2,2-Dimethyl-3-arylcyclopropancarbonsäureestern verwendet werden (vgl. „Coll. Czech. Chem. Commun.", *25* (1960), 1815).

*Beispiel 1*

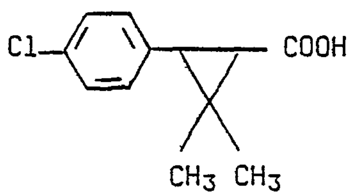

45 g (0,12 mol) 1-(4-Chlorphenyl)-1,4-dibrom-2,2-dimethyl-3-butanon werden bei 20 °C zu einer Lösung von 48,8 g (1,2 mol) Natriumhydroxid in 400 ml Wasser und 100 ml Dioxan tropfenweise gegeben. Das Reaktionsgemisch wird 20 h gerührt und mit Methylenchlorid extrahiert. Nach Ansäuern der wässerigen Phase wird erneut mit Methylenchlorid extrahiert. Nach Abdestillieren des Lösungsmittels von den vereinigten Extraktionslösungen erhält man 25,8 g (94% der Theorie) cis-/trans-2,2-Dimethyl-3-(4-chlorphenyl)cyclopropancarbonsäure vom Schmelzpunkt 115 °C.

Durch Umkristallisieren aus Diisopropylether erhält man die reine trans-2,2-Dimethyl-3-(4-chlorphenyl)cyclopropancarbonsäure vom Schmelzpunkt 137 °C.

*Beispiel 2*

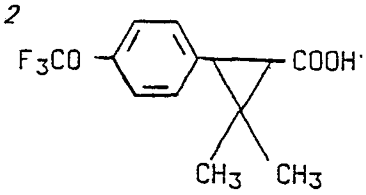

5,0 g 1,4-Dibrom-1-(4-trifluormethoxyphenyl)-2,2-dimethyl-3-butanon (Rohproduktherstellung siehe Beispiel 4, Gehalt ca. 85%) werden bei 20 °C zu einer Lösung von 4 g Natriumhydroxid in 40 ml Wasser und 20 ml Dioxan gegeben. Das Reaktionsgemisch wird 15 h gerührt und mit Methylenchlorid extrahiert. Die wässerige Phase wird angesäuert und erneut mit Methylenchlorid extrahiert. Nach Abdestillieren des Lösungsmittels von den vereinigten Extraktionslösungen erhält man 2,4 g 2,2-Dimethyl-3-(4-trifluormethoxyphenyl)-cyclopropancarbonsäure vom Schmelzpunkt 86 °C.

*Beispiel 3*

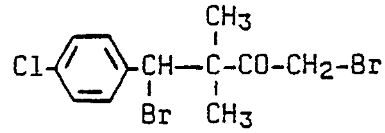

54 g (0,187 mol) 1-(4-Chlorphenyl)-1-brom-2,2-dimethyl-3-butanon werden in 500 ml Chloroform gelöst. Bei einer Innentemperatur von 5 °C wird eine Lösung von 29,9 g (0,187 mol) Brom in 100 ml Chloroform zugetropft. Dann lässt man das Reaktionsgemisch unter Rühren auf Raumtemperatur kommen und destilliert das Lösungsmittel unter vermindertem Druck ab. Man erhält 66 g eines Öls, das hauptsächlich aus 1-(4-Chlorphenyl)-1,4-dibrom-2,2-dimethyl-3-butanon steht.

$^1H$-NMR ($CDCl_3$): $\delta$ = 1,2 und 1,55 ppm (6H, 2 $CH_3$)
4,15 ppm ($CH_2$)
5,35 ppm (CH)
7,35 ppm (4H, Ar-H)

*Beispiel 4*

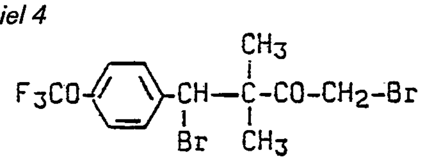

1,4-Dibrom-1-(4-trifluormethoxyphenyl)-2,2-dimethyl-3-butanon wurde analog Beispiel 3 als Rohprodukt erhalten und ohne weitere Reinigung weiter umgesetzt (vgl. Beispiel 2).

*Beispiel 5*

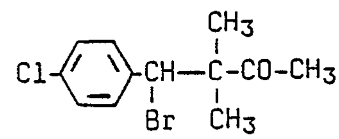

50 g (0,237 mol) 1-(4-Chlorphenyl)-2,2-dimethyl-3-butanon werden in 500 ml Tetrachlormethan gelöst und 44,5 g (0,25 mol) N-Bromsuccinimid sowie 0,5 g Azobisisobutyronitril dazugegeben. Das Reaktionsgemisch wird unter Rückfluss zum Sieden erhitzt, bis beim Abstellen des Rührers keine feste Substanz mehr zum Boden sinkt. Nach dem Abkühlen wird vom Succinimid abgesaugt, vom Filtrat das Lösungsmittel im Wasserstrahlvakuum abdestilliert und der Rückstand im Hochvakuum destilliert.

Man erhält 56,3 g (82% der Theorie) 1-(4-Chlorphenyl)-1-brom-2,2-dimethyl-3-butanon vom Siedepunkt 112-118 °C/0,2 mbar.

*Beispiel 6*

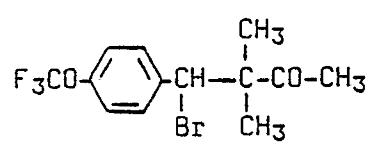

1-(4-Trifluormethoxyphenyl)-1-brom-2,2-dimethyl-3-butanon wurde analog Beispiel 5 erhalten. Siedepunkt 95-104 °C/0,2 mbar; $n_D^{20}$: 1,502.

*Beispiel 7*

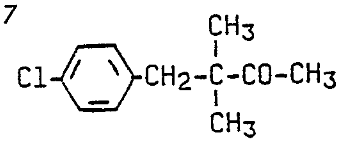

254 g (4 mol) pulverisiertes technisches Kaliumhydroxid (88%ig) werden in 1 l Toluol suspendiert. Hierzu werden 40 g Tetrabutylammoniumbromid und ein Gemisch von 644 g (4 mol) 4-Chlorbenzylchlorid und 430 g (5 mol) Methylisopropylketon bei 85 °C langsam gegeben. Das Reaktionsgemisch wird noch 3 h bei 85 °C gerührt; nach dem Abkühlen wird filtriert und das Filtrat neutral gewaschen. Durch fraktionierte Hochvakuumdestillation erhält man 732,5 g (87% der Theorie) 1-(4-Chlorphenyl)-2,2-dimethyl-3-butanon vom Siedepunkt 87-90 °C/0,05 mbar.

*Beispiel 8*

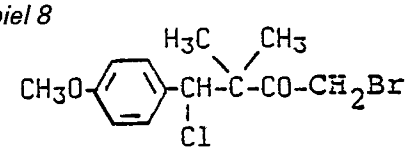

20 g (0,083 mol) 2,2-Dimethyl-1-chlor-1-(4-methoxyphenyl)-3-butanon („Archiv der Pharmazie", *308*, 422) werden in 150 ml Chloroform gelöst und bei Raumtemperatur 13,28 g (0,083 mol) Brom zugetropft. Man rührt 2 h bei 20-25 °C nach und destilliert das Lösungsmittel ab. Es bleiben 26,5 g eines Öls zurück, das hauptsächlich aus 4-Brom-2,2-dimethyl-1-chlor-1-(4-methoxyphenyl)-3-butanon besteht und direkt weiter umgesetzt wird.

*Beispiel 9*

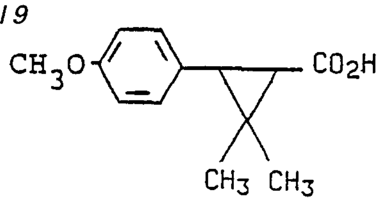

Zur Lösung von 35,3 g (0,882 mol) Natriumhydroxid in 317 ml Wasser gibt man 100 ml Aceton. Dann werden bei 25 °C 26,5 g (0,083 mol) 4-Brom-2,2-dimethyl-1-chlor-1-(4-methoxyphenyl)-3-butanon getropft.

Man rührt 12 h bei 25 °C nach, giesst auf Wasser und extrahiert mit Methylenchlorid. Die Wasserphase wird angesäuert und erneut extrahiert. Man erhält 12,6 g (69% der Theorie) 2,2-Dimethyl-3-(4-methoxyphenyl)cyclopropancarbonsäure vom Schmelzpunkt 115 °C.

## Patentansprüche

1. Verfahren zur Herstellung von 2,2-Dimethyl-3-arylcyclopropancarbonsäuren (-carbonsäureestern) der Formel (I)

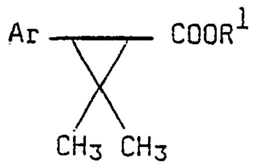

(I)

in welcher

$R^1$ für H oder $C_1$-$C_4$-Alkyl steht, und

Ar für Naphthyl oder für den Rest

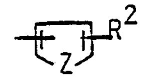

steht,

worin

Z für Sauerstoff, Schwefel oder Ethen-1,2-diyl (–CH=CH–) steht,

$R^2$ für Wasserstoff, Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht,

dadurch gekennzeichnet, dass man 1-Aryl-1,4-dihalogen-2,2-dimethyl-3-butanone der Formel (II)

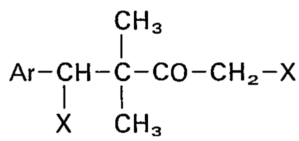

(II)

in welcher

Ar die oben angegebene Bedeutung hat, und

X für Chlor oder Brom steht,

mit Basen aus der Reihe der Alkalimetall- und Erdalkalimetallhydroxide, -alkoholate und -carbonate in Gegenwart von Wasser und organischer Lösungsmittel gegebenenfalls in Gegenwart von Katalysatoren bei Temperaturen zwischen −20 und +150 °C umsetzt.

2. 1-Aryl-1,4-dihalogen-2,2-dimethyl-3-butanone der Formel (II)

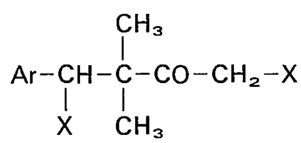

(II)

in welcher

X für Chlor oder Brom steht, und

Ar für Naphthyl oder für den Rest

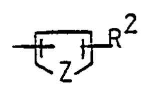

steht,

worin

Z für Sauerstoff, Schwefel oder Ethen-1,2-diyl (–CH=CH–) steht, und

$R^2$ für Wasserstoff, Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht.

3. Verfahren zur Herstellung der neuen 1-Aryl-1,4-dihalogen-2,2-dimethyl-3-butanone der Formel (II) gemäss Anspruch 2, dadurch gekennzeichnet, dass man 1-Aryl-1-halogen-2,2-dimethyl-3-butanone der Formel (III)

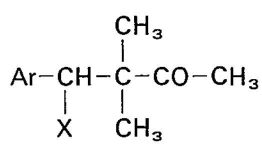

(III)

in welcher

Ar und X die in Anspruch 2 angegebene Bedeutung haben, mit Halogenen (Chlor oder Brom) in Gegenwart inerter Verdünnungsmittel bei Temperaturen zwischen −30 und +50 °C umsetzt.

4. 1-Aryl-1-halogen-2,2-dimethyl-3-butanone der Formel (IIIa)

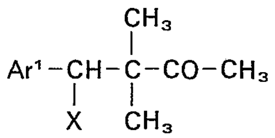 (IIIa)

in welcher

X für Chlor oder Brom steht, und

$Ar^1$ für Naphthyl oder für den Rest

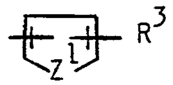

steht,

worin

$Z^1$ für Sauerstoff oder Ethen-1,2-diyl (−CH=CH−) steht, und

$R^3$ für Cyano, Nitro, Trialkylsilyl oder für einen gegebenenfalls durch Halogen substituierten Rest aus der Reihe Cycloalkyl, Alkenyl, Alkylendioxy, Alkylthio, Alkylsulfinyl, Alkylsulfonyl, Dialkylamino, Phenyl und Phenoxy, oder für von Methyl verschiedenes Alkyl, oder für von Methoxy verschiedenes Alkoxy, oder für Halogenalkyl oder Halogenalkoxy steht, oder, für den Fall, dass X für Brom steht, auch für Wasserstoff, Halogen, Methyl oder Methoxy steht.

5. Verfahren zur Herstellung der neuen 1-Aryl-1-halogen-2,2-dimethyl-3-butanone der Formel (IIIa) gemäss Anspruch 4, dadurch gekennzeichnet, dass man 1-Aryl-2,2-dimethyl-3-butanone der Formel (IV)

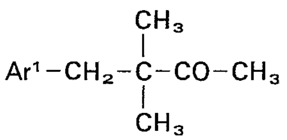 (IV)

in welcher

Ar die in Anspruch 4 angegebene Bedeutung hat, gegebenenfalls in Gegenwart eines inerten Verdünnungsmittels und in Gegenwart eines Katalysators bei Temperaturen zwischen 40 und 120 °C radikalisch halogeniert.

## Claims

1. Process for the preparation of 2,2-dimethyl-3-arylcyclopropanecarboxylic acids (carboxylic esters) of the Formula (I)

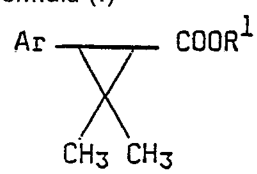 (I)

in which

$R^1$ represents H or $C_1$-$C_4$-alkyl, and

Ar represents naphthyl or the radical

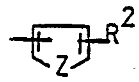

wherein

Z represents oxygen, sulphur or 1,2-ethenediyl (−CH=CH−),

$R^2$ represents hydrogen, halogen, cyano, nitro, trialkylsilyl or a radical, which is optionally substituted by halogen, from the series comprising alkyl, cycloalkyl, alkenyl, alkoxy, alkylenedioxy, alkylthio, alkylsulphinyl, alkylsulphonyl, dialkylamino, phenyl and phenoxy,

characterised in that 1-aryl-1,4-dihalogeno-2,2-dimethyl-3-butanones of the Formula (II)

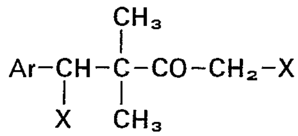 (II)

in which

Ar has the abovementioned meaning, and

X represents chlorine or bromine,

are reacted with bases from the series comprising the alkali metal and alkaline earth metal hydroxides, alcoholates and carbonates, in the presence of water and organic solvents, where appropriate in the presence of catalysts, at temperatures between −20 and +150 °C.

2. 1-Aryl-1,4-dihalogeno-2,2-dimethyl-3-butanones of the Formula (II)

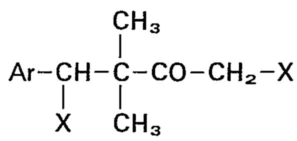 (II)

in which

X represents chlorine or bromine, and

Ar represents naphthyl or the radical

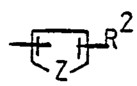

wherein

Z represents oxygen, sulphur or 1,2-ethenediyl (−CH=CH−), and

$R^2$ represents hydrogen, halogen, cyano, nitro, trialkylsilyl or a radical, which is optionally substituted by halogen, from the series comprising alkyl, cycloalkyl, alkenyl, alkoxy, alkylenedioxy, alkylthio, alkylsulphinyl, alkylsulphonyl, dialkylamino, phenyl and phenoxy.

3. Process for the preparation of the new 1-aryl-1,4-dihalogeno-2,2-dimethyl-3-butanones of the Formula (II) according to Claim 2, characterised in that 1-aryl-1-halogeno-2,2-dimethyl-3-butanones of the Formula (III)

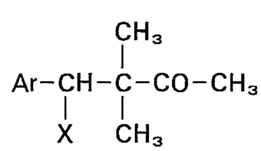 (III)

in which

Ar and X have the meaning given in Claim 2, are reacted with halogens (chlorine or bromine) in the

presence of inert diluents at temperatures between −30 and +30°C.

4. 1-Aryl-1-halogeno-2,2-dimethyl-3-butanones of the Formula (IIIa)

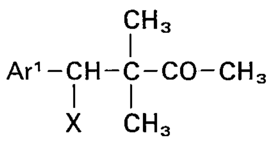

(IIIa)

in which

X represents chlorine or bromine, and

$Ar^1$ represents naphthyl or the radical

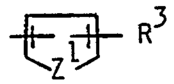

wherein

$Z^1$ represents oxygen or 1,2-ethenediyl (−CH=CH−), and

$R^3$ represents cyano, nitro, trialkylsilyl or a radical, which is optionally substituted by halogen, from the series comprising cycloalkyl, alkenyl, alkylenedioxy, alkylthio, alkylsulphinyl, alkylsulphonyl, dialkylamino, phenyl and phenoxy, or represents alkyl which is different from methyl, or represents alkoxy which is different from methoxy, or represents halogenoalkyl or halogenoalkoxy, or, in the case where X represents bromine, also represents hydrogen, halogen, methyl or methoxy.

5. Process for the preparation of the new 1-aryl-1-halogeno-2,2-dimethyl-3-butanones of the Formula (IIIa) according to Claim 4, characterised in that 1-aryl-2,2-dimethyl-3-butanones of the Formula (IV)

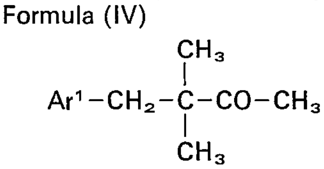

(IV)

in which

Ar has the meaning given in Claim 4,

are subjected to radical halogenation, where appropriate in the presence of an inert diluent and in the presence of a catalyst at temperatures between 40 and 120°C.

**Revendications**

1. Procédé de préparation d'acides 2,2-diméthyl-3-arylcyclopropanecarboxyliques (esters d'acides carboxyliques de formule (I)

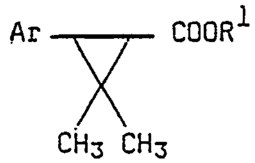

(I)

dans laquelle

$R^1$ représente H ou un groupe alkyle en $C_1$-$C_4$, et

Ar représente un groupe naphtyle ou le reste

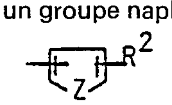

dans lequel

Z représente l'oxygène, le soufre ou un groupe éthène-1,2-diyle (−CH=CH−), et

$R^2$ représente l'hydrogène, un halogène, un groupe cyano, nitro, trialkylsilyle ou un reste, éventuellement substitué par des halogènes, de la série alkyle, cycloalkyle, alcényle, alcoxy, alkylènedioxy, alkylthio, alkylsulfinyle, alkylsulfonyle, dialkylamino, phényle et phénoxy,

caractérisé en ce que l'on fait réagir des 1-aryl-1,4-dihalogéno-2,2-diméthyl-3-butanones de formule (II)

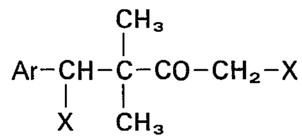

(II)

dans laquelle

Ar a les significations indiquées ci-dessus, et

X représente le chlore ou le brome,

avec des bases de la série des hydroxydes, alcoolates et carbonates de métaux alcalins et alcalino-terreux, en présence d'eau et de solvants organiques, éventuellement en présence de catalyseurs, à des températures de −20 à +150°C.

2. Les 1-aryl-1,4-dihalogéno-2,2-diméthyl-3-butanones de formule (II)

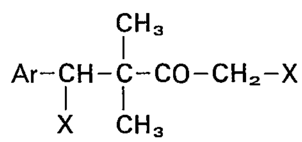

(II)

dans laquelle

X représente le chlore ou le brome, et

Ar représente un groupe naphtyle ou le reste

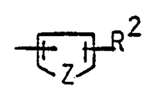

dans lequel

Z représente l'oxygène, le soufre ou un groupe éthène-1,2-diyle (−CH=CH−), et

$R^2$ représente l'hydrogène, un halogène, un groupe cyano, nitro, trialkylsilyle, ou un reste éventuellement substitué par des halogènes, de la série alkyle, cycloalkyle, alcényle, alcoxy, alkylènedioxy, alkylthio, alkylsulfinyle, alkylsulfonyle, dialkylamino, phényle et phénoxy.

3. Procédé de préparation des nouvelles 1-aryl-1,4-dihalogéno-2,2-diméthyl-3-butanones de formule II selon la revendication 2, caractérisé en ce que l'on fait réagir des 1-aryl-1-halogéno-2,2-diméthyl-3-butanones de formule (III)

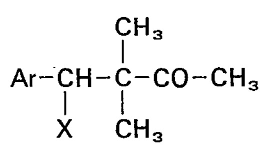

(III)

dans laquelle

Ar et X ont les significations indiquées dans la revendication 2, avec des halogènes (chlore ou brome) en présence de diluants inertes, à des températures de −30 à +50°C.

4. Les 1-aryl-1-halogéno-2,2-diméthyl-3-butanones de formule (IIIa)

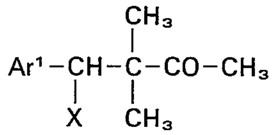 (IIIa)

dans laquelle

X représente le chlore ou le brome, et

$Ar^1$ représente un groupe naphtyle ou le reste

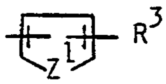

dans lequel

$Z^1$ représente l'oxygène ou le groupe éthène-1,2-diyle ($-CH=CH-$), et

$R^3$ représente un groupe cyano, nitro, trialkylsilyle ou un reste, éventuellement substitué par des halogènes, de la série cycloalkyle, alcényle, alkylènedioxy, alkylthio, alkylsulfinyle, alkylsulfonyle, dialkylamino, phényle et phénoxy, ou alkyle autre que méthyle, ou alcoxy autre que méthoxy, ou halogénoalkyle ou halogénoalcoxy, ou bien encore, lorsque X représente le brome, l'hydrogène, un halogène, un groupe méthyle ou méthoxy.

5. Procédé de préparation des nouvelles 1-aryl-1-halogéno-2,2-diméthyl-3-butanones de formule (IIIa) selon la revendication 4, caractérisé en ce que l'on soumet des 1-aryl-2,2-diméthyl-3-butanones de formule (IV)

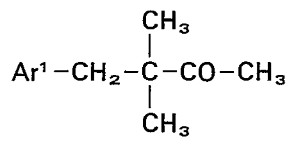 (IV)

dans laquelle Ar a les significations indiquées dans la revendication 4,

éventuellement en présence d'un diluant inerte et en présence d'un catalyseur, à des températures de 40 à 120 °C, à une halogénation radicalaire.